Europäisches Patentamt

European Patent Office

Office européen des brevets

(19)

(11) Numéro de publication: **0 243 249**
**A1**

(12) **DEMANDE DE BREVET EUROPEEN**

(21) Numéro de dépôt: **87400871.7**

(22) Date de dépôt: **16.04.87**

(51) Int. Cl.³: **A 61 M 5/20**
**F 16 K 3/00**

(30) Priorité: **18.04.86 FR 8605611**
**18.04.86 FR 8605612**

(43) Date de publication de la demande:
**28.10.87 Bulletin 87/44**

(84) Etats contractants désignés:
**AT BE CH DE ES GB IT LI LU NL SE**

(71) Demandeur: **Carre, Maurice**
**47, rue Alfred de Musset Chemin Vert**
**F-14000 Caen(FR)**

(72) Inventeur: **Carre, Maurice**
**47, rue Alfred de Musset Chemin Vert**
**F-14000 Caen(FR)**

(74) Mandataire: **Orès, Bernard et al,**
**Cabinet ORES 6, Avenue de Messine**
**F-75008 Paris(FR)**

(54) Dispositif de distribution, notamment à usage médical, d'un produit suivant des quantités dosées prédéterminées.

(57) Dispositif de distribution d'un produit suivant des quantités dosées, prédéterminées, dans lequel le produit à distribuer est enfermé dans un réservoir comprenant un orifice de sortie et auquel sont associés des moyens tendant en permanence à provoquer la sortie du produit hors dudit réservoir.

Il comprend sur le trajet de sortie du produit hors dudit réservoir (10) deux valves ou clapets (30, 32 – 31, 34) adjacents comportant chacun un ajutage calibré propre à être ouvert et/ou fermé en correspondance chacun de la condition d'un cristal piézo-électrique (33, 35) déformable et des moyens électroniques de commande de la déformation desdits cristaux pour limiter la sortie de produit hors dudit réservoir et ainsi définir une quantité dosée, prédéterminée.

FIG.1

1

L'invention a pour objet un dispositif de distribution, notamment à usage médical, d'un produit suivant des quantités dosées prédéterminées.

C'est, d'une façon générale, un but de l'invention de fournir un dispositif de distribution d'un produit sous forme liquide ou gazeuse suivant des quantités dosées prédéterminées, qui soit d'une grande fiabilité et, de ce fait, qui puisse être incorporé à des appareils de dosage de précision, notamment des appareils à usage médical.

L'invention vise en particulier, et sans que cette indication ait quelque caractère limitatif que ce soit, un tel dispositif propre à être utilisé pour délivrer suivant des quantités précises et prédéterminées un produit pharmaceutique à usage humain ou vétérinaire, de traitement ou de diagnostic, en particulier par injection parentérale.

On sait que le traitement de certaines maladies requiert des injections fréquentes de médicament, par exemple des injections journalières d'insuline dans le cas de certaines formes de diabète. Les patients qui souffrent de ces maladies doivent avoir recours aux soins de médecins, d'infirmiers ou effectuer eux-mêmes les injections nécessaires. L'emploi de seringues, même du type jetable, constitue une gêne pour l'utilisateur. En outre, les manipulations et préparations nécessaires pour l'exécution des injections à l'aide de telles seringues accroissent encore les désagréments inhérents à de tels traitements.

On a déjà proposé des dispositifs cherchant à éviter une partie des inconvénients rappelés ci-dessus lors des traitements comportant des injections répétées de substance médicamenteuse ou analogue et l'on peut se référer, à cet égard, à FR-A-2 348 709 ou US-A-4 345 595.

Dans l'un et l'autre de ces documents, une aiguille d'injection forme l'extrémité d'une seringue à piston mobile, l'amplitude de déplacement du piston déterminant le volume injecté. Si celui-ci doit être déterminé avec précision

2

les moyens mécaniques de commande deviennent complexes, lourds et coûteux de sorte que le dispositif n'est plus bien approprié pour une utilisation de traitements fréquents.

C'est, par conséquent, un but de l'invention de fournir un dispositif dont l'emploi soit plus simple que celui des dispositifs actuellement connus pour l'exécution de piqûres percutanées ou intramusculaires, et tels que définis, par exemple, dans les documents mentionnés ci-dessus.

C'est, encore, un but de l'invention de fournir un tel dispositif dont le fonctionnement automatique ou semi-automatique soit à la fois simple et sûr comme requis pour des appareils à usage de médecine humaine ou animale.

Sous un premier aspect, un dispositif selon l'invention, dans lequel le produit à distribuer est enfermé dans un réservoir comprenant un orifice de sortie et auquel sont associés des moyens tendant en permanence à provoquer la sortie du produit hors dudit réservoir, est caractérisé en ce qu'il comprend, sur le trajet de sortie du produit hors dudit réservoir, deux valves ou clapets adjacents comportant chacun un ajutage calibré propre à être ouvert et/ou fermé en correspondance chacun de la condition d'un cristal piézo-électrique déformable et des moyens électroniques de commande de la déformation desdits cristaux pour limiter la sortie de produit hors dudit réservoir et ainsi définir une quantité dosée, prédéterminée.

En raison de la présence des deux valves ou clapets à commande piézo-électrique, un dispositif selon l'invention est d'une grande sécurité de fonctionnement, de sorte qu'il peut trouver application dans des domaines nécessitant une très grande fiabilité comme requis en médecine humaine, par exemple pour l'injection d'insuline.

Dans une forme de réalisation, le réservoir de produit à distribuer est un réservoir souple ou un système à piston et les moyens qui tendent à provoquer la sortie du produit à distribuer hors dudit réservoir sont des moyens qui commandent la déformation dudit réservoir ou font déplacer le piston.

Dans une forme de réalisation particulièrement avantageuse, lesdits moyens sont constitués par l'effet de pression, variable en fonction de la température, de la vapeur d'un gaz, comme un fréon choisi de manière que ses caractéristiques de tension de vapeur soient compatibles avec la puissance maximum admissible des cristaux piézo-électriques mis en oeuvre.

A cet égard, une réalisation prévoit que ledit gaz soit du fréon 13.

Selon une autre caractéristique de l'invention, le dispositif est muni de moyens propres à déclencher un signal sonore ou lumineux d'alarme pour des conditions préétablies de fonctionnement, par exemple lorsqu'est atteint un certain niveau minimal dans la réserve de produit à délivrer, ou en cas de défaillance du circuit électronique de commande, ou encore en cas de mauvais fonctionnement des moyens assurant la sortie de produit à délivrer hors du réservoir qui le contient.

Dans un mode de réalisation, la déformation d'un des cristaux piézo-électrique est commandée par l'intermédiaire de la tension fournie par un convertisseur continu-continu dont une entrée est reliée à un comparateur lequel reçoit d'une part une tension de référence et, d'autre part, un signal de déclenchement.

Dans ce mode de réalisation, les moyens électroniques de commande comprennent en outre un premier amplificateur dont une entrée est reliée à une borne d'un premier cristal piézo-électrique ayant son autre borne à la masse et dont la sortie est reliée à l'une des bornes d'un condensateur dont le courant de charge est réglable par l'intermédiaire d'une résistance variable pour la calibration initiale.

Un second amplificateur dont une entrée est reliée audit premier cristal piézo-électrique et dont la sortie traduit l'état dudit cristal aboutit au convertisseur continu-continu.

Pour déterminer la quantité dosée, prédéterminée que délivre le dispositif, celui-ci comprend en outre des moyens de base de temps pour le comptage du nombre d'impulsions de déclenchement appliquées au comparateur.

De façon avantageuse, les cristaux piézo-électriques sont protégés à l'encontre de l'action du produit à distribuer par une enveloppe protectrice en un matériau comme une résine souple polymérisable, ladite enveloppe s'opposant simultanément à des fuites électriques susceptibles de détériorer, par électrolyse ou de façon analogue, le produit à distribuer.

Sous un second aspect, l'invention vise des réalisations de dispositifs d'injection de liquide à usage médical, comportant une aiguille d'injection et un réservoir de produit à injecter relié de façon opératoire à ladite aiguille, avec des moyens interposés entre ledit réservoir et ladite aiguille pour la commande de débit du produit injecté, caractérisé en ce que lesdits moyens sont constitués par un dispositif piézo-électrique comprenant deux valves ou clapets adjacents comportant chacun un ajutage calibré propre à être ouvert/ou fermé en correspondance chacun de la condition d'un cristal piézo-électrique déformable, et des moyens électroniques de commande de la déformation desdits cristaux pour limiter la sortie du produit hors dudit réservoir et, ainsi, définir une quantité dosée, prédéterminée.

L'invention envisage des réalisations différant entre elles par leur mode d'utilisation, par exemple en tant que dispositif portable et rechargeable après exécution d'un certain nombre d'injections et/ou épuisement de la réserve de produit à injecter, ou en tant que dispositif pour l'injection parentérale par voie sous cutanée ou intra-musculaire de substance médicamenteuse sur une période de temps relativement longue, comme requis, par exemple pour certains traite-

5

ments continus prolongés dans le cas de pompes ambulatoires ou analogues.

L'invention sera bien comprise par la description qui suit, faite à titre d'exemple et en référence au dessin annexé dans lequel :

- la figure 1 est une vue schématique d'un dispositif selon l'invention ;

- la figure 2 est un schéma électronique ;

- la figure 3 montre une forme d'onde mise en oeuvre dans un dispositif selon l'invention ;

- la figure 4 est une vue partielle, à plus grande échelle, d'une partie du dispositif montré sur la figure 1 ;

- la figure 5 est une vue de dessus correspondante, certaines parties ayant été écartées des autres dans un but de clarté ;

- la figure 6 est une vue de côté dans laquelle certaines parties ont également été écartées les unes des autres dans un but de clarté ;

- la figure 7 est une vue schématique, en coupe longitudinale, d'un dispositif d'injection de liquide à usage médical.

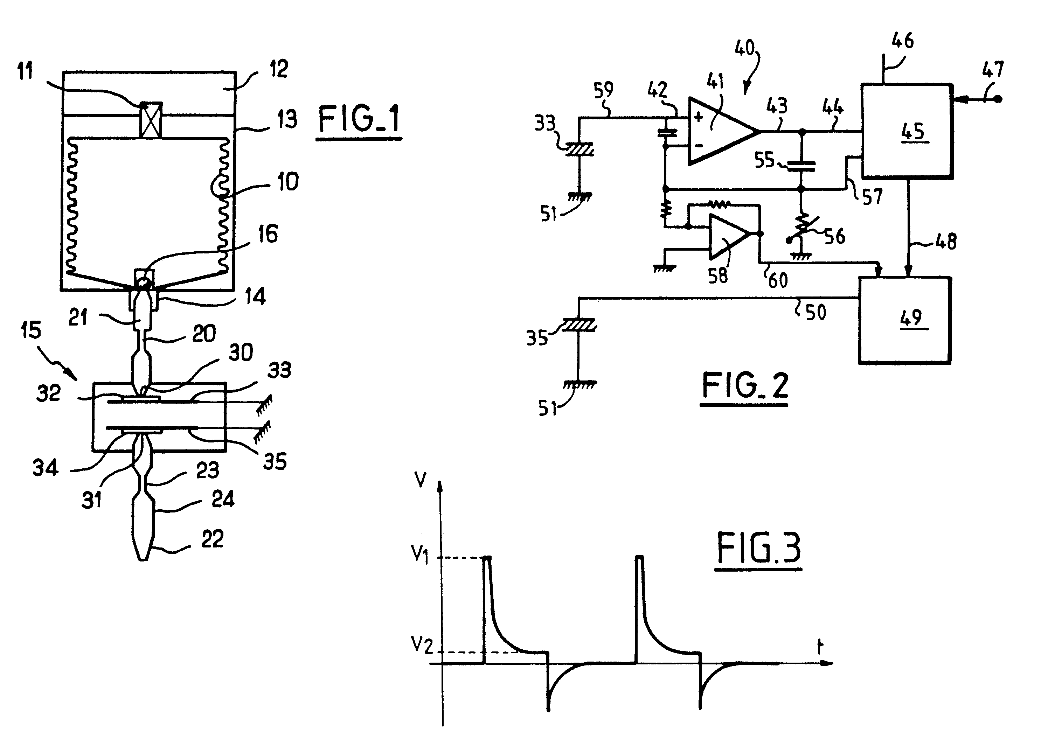

Un dispositif selon l'invention, distributeur d'un produit suivant des quantités dosées, prédéterminées, est montré schématiquement sur la figure 1. Il comprend un réservoir 10 de produit à distribuer, auquel sont reliés de façon opératoire comme montré schématiquement en 11, des moyens 12 qui tendent en permanence à commander la sortie hors dudit réservoir du produit qu'il contient.

Dans la forme de réalisation décrite et représentée, le réservoir 10 est avantageusement un réservoir souple, déformable, enfermé dans une enceinte 13, laquelle enferme également les moyens 12, constitués de préférence par une réserve de gaz, - comme un fréon -, dont la tension de vapeur, à la température d'utilisation du dispositif, est choisie

6

pour l'obtention d'une force de poussée appropriée, provoquant l'éjection hors du réservoir 10 du produit qu'il contient par déformation dudit réservoir. L'enceinte 13 est également organisée de manière à ce que l'on puisse procéder aisément, à volonté, au remplacement soit de la réserve de gaz 12, soit du réservoir souple de produit à distribuer, ledit réservoir étant en outre muni d'un moyen 16, comme une soupape ou analogue à fermeture étanche, destiné à permettre l'introduction initiale dans la réserve 10 du produit à distribuer, mais qui ne s'oppose pas à sa sortie.

Dans une variante, non représentée, le produit à distribuer est enfermé dans un système à cylindre et piston.

A l'ensemble de réservoir tel que décrit ci-dessus est associé un dispositif 15 de dosage proprement dit, interposé entre le réservoir 10 de produits à distribuer auquel il est relié par un capillaire 20 et un embout de sortie 22 auquel il est relié par un autre capillaire 23. Le capillaire 20 est interposé sur une canalisation 21 de liaison du dispositif de dosage proprement dit 15 et du réservoir 10, tandis que le capillaire 23 est interposé sur une canalisation 24 reliant la sortie du dispositif 15 de dosage proprement dit à l'empout 22.

Le dispositif 15 comprend un premier ajutage calibré ou buse 30 communiquant directement avec le capillaire 20 et un second ajutage calibré ou buse 31 communiquant avec le capillaire 23, lesdites buses étant avantageusement réalisées en une matière plastique du type Nylon (marque déposée) injectée ou en acier inoxydable usiné et fileté à un pas micrométrique.

Avec l'orifice de sortie de la buse 30 est propre à coopérer un organe 32 porté par un cristal piézo-électrique 33 dont la précontrainte amène ledit organe 32 au contact dudit orifice pour en assurer la fermeture, tandis que le produit à distribuer s'échappe au travers dudit orifice lorsque l'organe 32 est écarté par le produit sous pression en déformant

7

le cristal piézo-électrique, provoquant ainsi l'apparition d'une charge électrique à ses bornes. De façon analogue, avec l'orifice de sortie calibré de la buse 31 coopère un organe 34 porté par un cristal piézo-électrique 35 dont le mouvement ouvre ou ferme à étanchéité l'orifice de la buse 31, l'effort d'ouverture des clapets que forme ladite buse 31 et l'organe 34 étant limité par le diamètre du capillaire 23, lequel limite également le débit de sortie de produit à distribuer.

Selon l'invention, les cristaux piézo-électriques 33 et 35 sont commandés par un circuit électronique 40, figure 2, lequel comprend un amplificateur 41 dont une entrée 42 est reliée à une borne 59 du cristal 33 et dont la sortie 43 est reliée à une entrée 44 d'un comparateur 45 lequel reçoit sur une borne 46 une tension de référence et sur une autre entrée 47 un signal de déclenchement. La sortie 48 du comparateur 45 est reliée à un convertisseur continu-continu 49 dont la sortie 50 est reliée à une borne du cristal piézo-électrique 35, l'autre borne de celui-ci étant reliée à la masse 51.

En dérivation sur la sortie 43 de l'amplificateur 41 est prévu un condensateur 55 dont le courant de charge est réglable par une résistance variable 56, la tension aux bornes de ladite capacité 55 étant appliquée au comparateur 45 par une borne d'entrée 57.

Un second amplificateur 58, également relié à la borne 59 du cristal piézo-électrique 33, fournit sur sa sortie 60, - reliée au convertisseur 49 -, une image de l'état dudit cristal.

Le fonctionnement d'un dispositif selon l'invention est le suivant :

Le réservoir 10 étant rempli de produit et les moyens 12 étant opératoires, le produit à distribuer tend à sortir hors dudit réservoir.

Dans une condition initiale qui est celle montrée

8

schématiquement sur la figure 1, l'orifice de sortie de la buse 30 est obturé par coopération de contact à étanchéité de l'organe 32 et dudit orifice, de même qu'est fermé l'orifice de la buse 31 par coopération à étanchéité avec celle-ci de l'organe 34. Aucune tension n'existe aux bornes du cristal piézo-électrique 33 ou est appliquée aux bornes du cristal piézo-électrique 35 ; il n'y a pas distribution de produit à partir du réservoir 10.

Si, à partir de cette condition, une impulsion de déclenchement est appliquée sur la borne d'entrée 47 du comparateur 45, la sortie 48 de celui-ci bascule et provoque la mise en service du convertisseur 49. La tension croît alors aux bornes du cristal piézo-électrique 35 et, lorsque la tension aux bornes dudit cristal atteint une valeur $V_1$, - comme montré sur le diagramme de la figure 3 dans lequel on a porté en abscisses le temps et en ordonnées les valeurs de tension aux bornes du cristal piézo-électrique 35 -, ledit cristal se déforme de manière effective et rapide alors que la tension à ses bornes décroît pour être limitée à une valeur $V_2$ pour laquelle existe une déformation résiduelle suffisante pour ménager une ouverture de surface égale à la surface du capillaire 23 qui limite ainsi le débit maximum de produit, d'une part, et limite également, d'autre part, l'effort d'ouverture de la valve constituée par la buse 31 et l'organe 34.

Simultanément à la déformation du cristal piézo-électrique 35, qui commande l'ouverture de la buse 31, le cristal piézo-électrique 33 est également déformé par le passage du produit, ce qui provoque l'apparition sur sa borne 59 d'une tension qui, appliquée par l'entrée 42 à l'amplificateur 41 avantageusement un amplificateur du type MOS FET à très haute impédance, produit un courant constant dont l'intensité, réglable par l'intermédiaire de la résistance 56, provoque la charge de la capacité 55, la tension qui apparaît aux bornes de celle-ci étant alors l'image de la quantité de produit libéré.

Lorsque cette tension atteint la valeur de la tension de référence appliquée sur l'entrée 46 du comparateur 45 la sortie 48 de ce dernier bascule et, la sortie du convertisseur 49 étant inversée ou ramenée à zéro, comme montré sur le diagramme de la figure 3, l'orifice de la buse 31 est fermé par retour du cristal 35 à sa condition initiale et maintenu fermé par la pression qui s'exerce sur le cristal piézo ce qui interdit toute nouvelle sortie du produit. Le retour simultané du cristal 33 à sa condition initiale, c'est-à-dire celle en laquelle l'organe 32 coopère avec la sortie de la buse calibrée 30, ramène à zéro la tension aux bornes du cristal piézo-électrique.

Au cours de cette étape la capacité 55 est également court-circuitée de sorte que l'apparition d'une nouvelle impulsion de déclenchement sur l'entrée 47 commande le déroulement d'un cycle identique à celui décrit ci-dessus et, par suite, la sortie hors du réservoir 10 d'une nouvelle quantité dosée, prédéterminée, de produit à distribuer.

On détermine alors la quantité de produit distribué, en réglant et en comptant, par des moyens non représentés, le nombre d'impulsions de déclenchement sur l'entrée 47 du comparateur 45.

La sortie de l'amplificateur 58, - qui est l'image de la condition du cristal piézo-électrique 33, c'est-à-dire de la condition de la valve que forme l'organe 32 porté par ledit cristal et la buse calibrée 30 -, commande au cours du cycle défini ci-dessus le passage de haute en moyenne tension et il fournit, en dehors des périodes correspondant à l'ouverture des valves du dispositif 15, une indication sur un éventuel débit résiduel au droit de l'orifice calibré de la buse 30, de sorte que sa sortie peut constituer le signal d'entrée d'un circuit d'alarme.

Le déclenchement de celle-ci peut avantageusement être sous la dépendance d'une deuxième base de temps, dont le démarrage est synchrone de celui de commande d'application des impulsions de déclenchement sur l'entrée 47 du comparateur 45.

L'invention prévoit aussi de provoquer le déclenchement d'une alarme en réponse à des facteurs comme l'absence de produit dans la réserve 10, une défaillance des moyens 12 tendant à provoquer l'éjection du produit, une défaillance des circuits électroniques ou de l'alimentation en énergie de ceux-ci.

Pour la protection des cristaux piézo-électriques 33 et 35 à l'encontre de l'action chimique ou physicochimique du produit distribué, l'invention prévoit de revêtir lesdits cristaux d'une résine polymérisable, par exemple une résine du type époxy souple, un tel traitement ayant complémentairement pour avantage d'éviter toute fuite électrique susceptible de détériorer le produit à distribuer, par exemple par électrolyse.

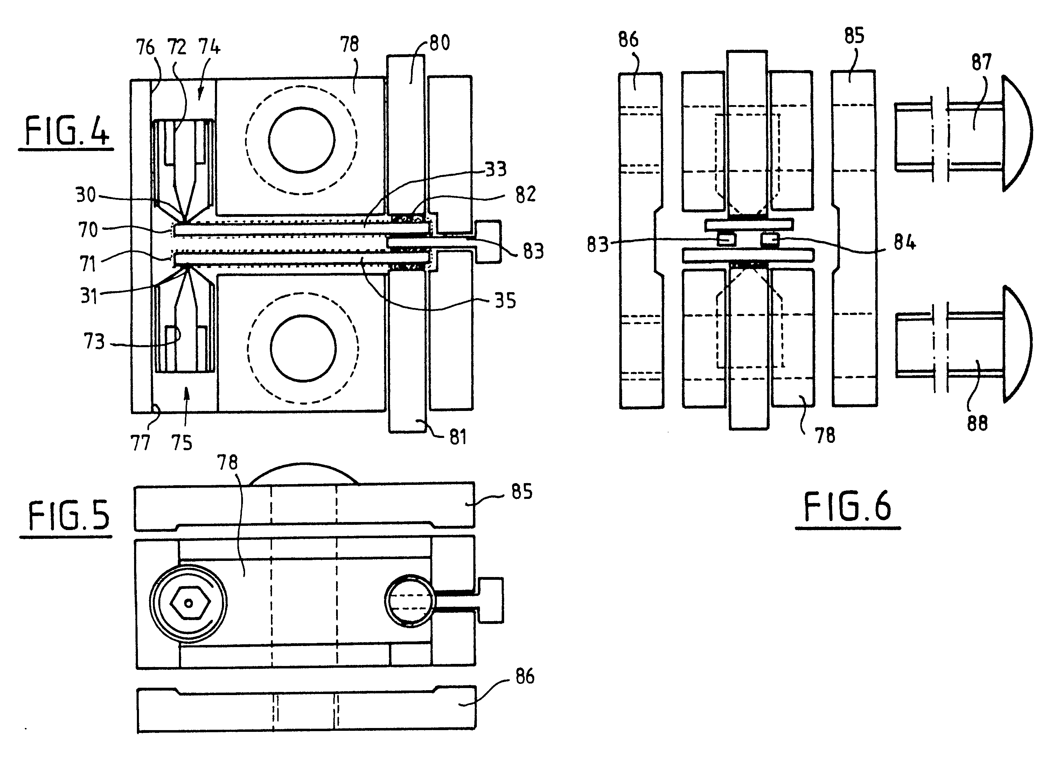

Comme montré sur les figures 4 à 6, les cristaux piézo-électriques 33 et 35, revêtus de film de résine 70 et 71, respectivement, coopèrent avec les buses 30 et 31 ménagées aux extrémités de forages longitudinaux 72 et 73 percés dans des pièces 74 et 75 de forme ogivale vissées et collées dans des forages cylindriques en regard 76 et 77 d'un corps 78 en matériau isolant. Dans celui-ci sont également montés des poussoirs 80 et 81 reliés électriquement à l'aide de zones de colle conductrice 82 et 83, respectivement, aux cristaux piézo-électriques 33 et 35 entre lesquels sont, en outre, interposés des écarteurs 83 et 84, également en relation de conduction électrique à l'aide d'une colle conductrice, par exemple une colle à l'argent, avec lesdits cristaux.

Avec le corps 78 coopèrent des couvercles 85 et 86 dont la fixation au corps est réalisée à l'aide de vis 87 et 88 et l'étanchéité à l'aide de joints, non représentés.

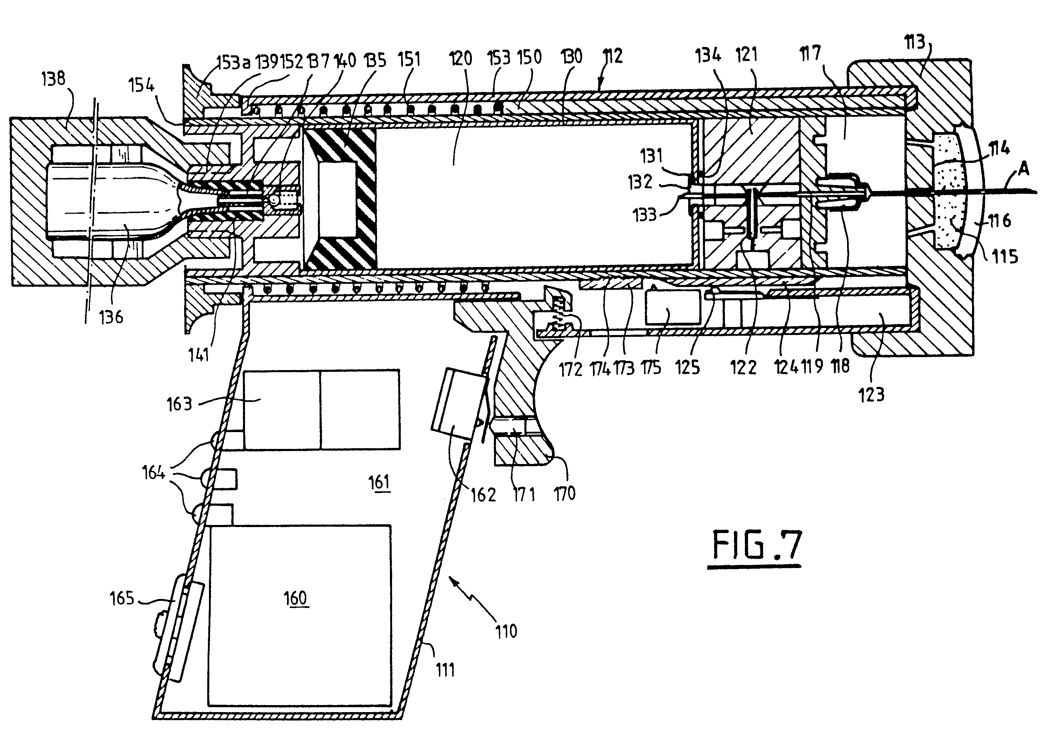

Pour la distribution par injection de liquide, suivant des quantités dosées, prédéterminées, par exemple d'insuline, on prévoit un dispositif 100 (figure 7) sous forme d'un pistolet à crosse 111 et canon 112 réalisé par assemblage de deux demi-coquilles montées sur des rails de guidage. Le canon 112 est obturé par un bouchon stérile 113 dans lequel est ménagé un logement 114 d'une pastille 115 de formol solide ou analogue maintenue en place par un bouchon hermétique 116. Dans l'espace stérile 117, que ferme le bouchon 113, fait saillie un organe 118 de connection d'une aiguille d'injection, non représentée, qui est percé d'un canal 119 de passage du produit à injecter délivré à partir d'une réserve 120 par un microdoseur 121 de contrôle et de commande du débit du produit à injecter et qui est du type de celui décrit ci-dessus en référence aux figures 1 à 6.

Ce dispositif, qui comprend des cristaux piézo-électriques 122, est commandé à partir de moyens électroniques, non représentés, placés dans un logement 123 du canon 112, la liaison électrique desdits moyens au microdoseur 121 étant réalisée par l'intermédiaire de plaques 124 d'une part, et de ressorts de contact 125, d'autre part.

Dans une première réalisation, représentée au dessin, la réserve 120 de produit à injecter est enfermée dans un cylindre 130 dont l'orifice de sortie est garni d'un moyen d'étanchéité en caoutchouc siliconé 132, lequel est traversé par une aiguille de percussion et de prélèvement du produit 133, un joint torique 134 étant interposé entre la face externe du cylindre 130 et le dispositif de microdoseur 121. Dans le cylindre 130 est logé un piston 135 en caoutchouc siliconé ou analogue sur lequel s'exerce l'effet de pression d'un gaz enfermé dans une cartouche 136, par exemple du fréon liquide, des moyens en soi connus 137 de percussion de la cartouche 136 étant opératoires lorsqu'une enveloppe 138 de la cartouche 136 est vissée sur un corps 139 qui enferme également un clapet anti-retour 140 et une bague d'étanchéité en caoutchouc 141.

Dans une variante, non représentée, le liquide à

12

injecté est enfermé dans un sac plastique souple logé dans le corps 130.

L'ensemble de l'aiguille d'injection A, du dispositif de dosage et de la réserve de produit à injecter est monté à coulissement dans des glissières du canon 112 du dispositif par l'intermédiaire de trois rails de guidage 150 équirépartis angulairement autour de l'axe du canon et sur lesquels s'exerce l'action d'un ressort 151 en butée à son extrémité arrière sur un arrêt 152 du corps 153 du canon et dont la position peut être ajustée à l'aide d'un anneau moleté 153a à filetage interne 154 coopérant avec le corps du dispositif.

Dans la crosse 111 sont logées les piles 160 d'alimentation électrique, les moyens électroniques de commande et de contrôle 161 et un microcontact de gâchette 162, un dispositif d'affichage à minidigits 163 ou analogue étant apparent sur la crosse, de même que des diodes électro-luminescentes 164 et un interrupteur de marche-arrêt 165.

La gâchette 170, qui comprend une vis 171 de réglage du microcontact 162 est soumise à l'action d'un ressort de rappel 172, une butée de gâchette 173, dont la position est réglable par une crémaillère 174, étant prévue sur le corps du canon 112, au voisinage d'un microcontact d'armement 175 dans la condition non armée du dispositif.

Le fonctionnement du dispositif est le suivant : après mise en marche à l'aide de l'interrupteur 165, mise en marche qui provoque la remise à zéro des moyens électroniques et la mise en service du doseur 121, on met en place l'aiguille d'injection A sur l'organe de connection 118 et on règle la longueur de sortie de ladite aiguille par action sur l'anneau moleté 153a.

Par une réalisation appropriée des circuits 161 l'actionnement de la gâchette 170 provoque un premier actionnement du microcontact 162 qui commande la purge de l'aiguille en maintenant le système électronique du microdoseur 121

13

bouclé sur lui-même et la libération d'une microdose de produit à injecter lors du relâchement de la gâchette 170.

On procède alors à l'armement du dispositif d'injection, par un mouvement vers l'arrière analogue à l'armement d'un pistolet. Cette action purement mécanique n'influence pas les moyens électroniques, à l'exception des diodes électro-luminescentes 164 dont l'une s'allume pour indiquer que le dispositif est armé. On affiche alors sur les moyens 163 la dose à injecter et on actionne à nouveau la gâchette 170 pour libérer la butée de gâchette 173 et provoquer le déplacement vers l'avant, sous l'action du ressort 151 précédemment comprimé, de l'ensemble mobile comportant l'aiguille d'injection, la réserve de liquide à injecter et le dispositif de dosage.

Celui-ci est simultanément rendu opératoire, comme décrit ci-dessus en référence aux figures 2 et 3 et, alors que la diode électro-luminescente d'armement s'éteint et que s'allume une diode électro-luminescente d'injection, ladite injection débute et se poursuit jusqu'à ce que la dose à injecter soit atteinte.

Un dispositif d'alarme qui, entre autres fonctions, reçoit les signaux du microdoseur 121 et prend également en compte la valeur de tension des piles 160, permet d'arrêter l'injection en cas de nécessité et/ou indique que les piles doivent être changées.

Un dispositif selon l'invention a été réalisé pour la distribution d'insuline en utilisant un réservoir souple à déformation commandée par l'action de la pression de vapeur d'un fréon 113, ayant une valeur de 8 kg/cm$^2$ à 30°C.

Le diamètre choisi pour les capillaires était de l'ordre de 72,5 µm, le diamètre des buses calibrées d'environ 1000 µm et celui des sièges coopérant avec lesdites buses était d'environ 2000 µm.

Le cristal piézo-électrique 33 était du type PXE5, bimorphe, d'une dimension de 12x4x0,6 mm, d'une lon-

14

gueur libre de 10 mm et revêtu, pour son isolation, d'une résine polymérisée souple, un tel cristal étant disponible auprès de la Société PHILIPS sous la référence 43 22 020 04 610.

Le cristal piézo-électrique 35, en le même matériau que celui du cristal piézo-électrique 33, avait des dimensions 12 x 6 x 0,6 mm et était disponible auprès de la Société PHILIPS sous la référence 43 22 020 04 650.

Pour un tel élément, la valeur de la tension de seuil est de l'ordre de 98 Volts et la valeur de la tension d'ouverture, pour une déflection de 8 µm de l'ordre de 32 Volts.

## REVENDICATIONS

1. Dispositif de distribution d'un produit suivant des quantités dosées, prédéterminées, dans lequel le produit à distribuer est enfermé dans un réservoir comprenant un orifice de sortie et auquel sont associés des moyens tendant en permanence à provoquer la sortie du produit hors dudit réservoir, caractérisé en ce qu'il comprend sur le trajet de sortie du produit hors dudit réservoir (10) deux valves ou clapets (30,32 - 31,34) adjacents comportant chacun un ajutage calibré propre à être ouvert et/ou fermé en correspondance chacun de la condition d'un cristal piézo-électrique (33, 35) déformable et des moyens électroniques de commande (40) de la déformation desdits cristaux pour limiter la sortie de produit hors dudit réservoir et ainsi définir une quantité dosée, prédéterminée.

2. Dispositif selon la revendication 1, caractérisé en ce que le réservoir de produit à distribuer est un réservoir souple (10) et en ce que les moyens provoquant la sortie du produit à distribuer hors dudit réservoir sont des moyens (12) qui commandent la déformation dudit réservoir (10).

3. Dispositif selon la revendication 2, caractérisé en ce que lesdits moyens (12) de déformation du réservoir (10) sont constitués par l'effet de pression, variable en fonction de la température, de la vapeur d'un gaz, comme un fréon, contenu dans une enveloppe montée de manière amovible dans une enceinte (13) qui loge également le réservoir (10) de produit à distribuer.

4. Dispositif selon la revendication 1, caractérisé en ce que la déformation d'un des cristaux piézo-électriques (35) est commandée par l'intermédiaire de la tension fournie par un convertisseur continu-continu (49) dont une entrée est reliée à un comparateur (45) lequel reçoit d'une part une tension de référence (46) et d'autre part un signal de déclenchement (47) et en ce que les moyens électroniques de commande (40) comprennent en outre un premier amplificateur (41) dont une entrée est reliée à une borne (59) d'un premier cristal piézo-électrique (33) ayant son autre borne à la masse (51) et dont la sortie (43) est reliée à l'une des bornes d'un condensateur (55) dont le courant de charge est réglable par l'intermédiaire d'une résistance variable (56).

5. Dispositif selon la revendication 4, caractérisé en ce qu'il comprend un second amplificateur (58) dont une entrée est reliée audit premier cristal piézo-électrique (33) et dont la sortie (60), qui traduit l'état dudit cristal, aboutit au convertisseur continu-continu (49).

6. Dispositif selon la revendication 4, caractérisé en ce qu'il comprend en outre des moyens de base de temps pour le comptage du nombre d'impulsions de déclenchement appliquées audit comparateur (45).

7. Dispositif selon la revendication 1, caractérisé en ce qu'il comprend un corps (78) en matériau isolant dans lequel sont montées des pièces (74, 75) ménageant les ajutages calibrés (30, 31), ledit corps présentant également un espace de réception des cristaux piézo-électriques (33, 35) sous forme de plaquettes dont le positionnement est assuré, simultanément à leurs liaisons électriques, par des poussoirs (80,81) logés dans le corps (78) et des écarteurs (83, 84) également logés dans ledit corps, les cristaux piézo-électriques (33, 35) étant protégés à l'encontre de l'action du produit à distribuer par une enveloppe protectrice en un matériau comme une résine souple polymérisable (70, 71), ladite enveloppe s'opposant simultanément à des fuites électriques susceptibles de détériorer, par électrolyse ou de façon analogue, le produit à distribuer.

8. Dispositif d'injection de liquide, notamment à usage médical, comportant une aiguille d'injection (A) et une réserve (120) de produit à injecter reliée de façon opératoire à ladite aiguille, avec des moyens piézo-électriques interposés entre ladite réserve et ladite aiguille pour la commande de débit du produit injecté, caractérisé en ce que lesdits moyens comprennent deux valves ou clapets adjacents comportant chacun un ajutage calibré propre à être ouvert/ou fermé en correspondance chacun de la condition d'un cristal piézo-électrique déformable (122) et des moyens électroniques (123) de commande de la déformation desdits cristaux pour limiter la sortie du produit hors dudit réservoir et, ainsi, définir une quantité dosée, prédéterminée.

9. Dispsoitif selon la revendication 8, caractérisé en ce qu'il est réalisé sous forme d'un pistolet dont la gâchette (170) commande l'injection par un mouvement de coulissement d'un ensemble comprenant un porte-aiguille d'injection (118), les moyens piézo-électriques et la réserve de produit (120), d'une part et, d'autre part, la mise en condition opératoire des moyens électroniques de contrôle et de commande du débit de liquide à injecter, après armement du pistolet et par actionnement d'un microcontact (162) avec lequel coopère la gâchette (170).

10. Dispositif selon la revendication 9, caractérisé en ce que la crosse (111) du dispositif en forme de pistolet comprend également un interrupteur de marche-arrêt (165), des moyens de visualisation de l'état du dispositif (164) et des moyens d'affichage des doses à injecter (163).

FIG.1

FIG.2

FIG.3

0243249

FIG.4

FIG.5

FIG.6

2/3

0243249

FIG.7

3/3

024 3249

Office européen
des brevets

## RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | US-A-4 340 083 (CUMMINS) <br> * Colonne 2, ligne 63 - colonne 7, ligne 68; figures * | 1,2,4 | A 61 M 5/20 <br> F 16 K 3/00 |
| Y | | 8 | |
| | --- | | |
| A | FR-A-2 370 481 (SIEMENS AG) <br> * Page 1, ligne 1 - page 2, ligne 4; page 4, ligne 3 - page 5, ligne 19; figure 1 * | 1-3 | |
| | --- | | |
| Y | US-A-4 345 595 (WHITNEY et al.) <br> * Colonne 2, lignes 51-55; figure 23 * | 8 | |
| | --- | | |
| A | FR-A-2 348 709 (M. PISTOR) <br> * Revendication 4; figure 1 * | 9-10 | DOMAINES TECHNIQUES RECHERCHES (Int Cl 4) |
| | --- | | A 61 M |
| A | US-A-3 152 612 (H. AVERY) <br> * Colonne 2, lignes 31-39; figure 3 * | 8 | F 16 K |
| | ----- | | |

Le présent rapport de recherche a été établi pour toutes les revendications

| Lieu de la recherche | Date d'achèvement de la recherche | Examinateur |
|---|---|---|
| LA HAYE | 08-07-1987 | EHRSAM F.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82